# FASCICULE DE BREVET EUROPEEN

(11) **EP 0 564 645 B1**
(45) Date de publication et mention de la délivrance du brevet: **29.12.1999**
(21) Numéro de dépôt: 92924753.4
(22) Date de dépôt: 30.10.1992
(51) Int. Cl.: A61K 48/00, C12N 5/00

(54) **CELLULES TRANSFORMEES POUR LA PREVENTION OU LE TRAITEMENT DE MALADIES INDUITES PAR DES VIRUS, NOTAMMENT RETROVIRUS PATHOGENES**
TRANSFORMIERTE ZELLEN ZUR VERHÜTUNG ODER ZUR BEHANDLUNG VON VIRUS-INDUZIERTEN ERKRANKUNGEN,BESONDERS PATHOGENEN RETROVIREN
TRANSFORMANT CELLS FOR THE PROPHYLAXIS OR TREATMENT OF DISEASES CAUSED BY VIRUSES, PARTICULARLY PATHOGENIC RETROVIRUSES

(30) Priorité: 30.10.1991 FR 9113429
(43) Date de publication de la demande: 13.10.1993
(73) Titulaire: UNIVERSITE PIERRE ET MARIE CURIE PARIS VI, 75252 Paris Cédex 05 (FR)
(72) Inventeur: KLATZMANN, David, F-75013 Paris (FR)
(74) Mandataire: Gutmann, Ernest
(86) Numéro de dépôt international: FR9201017
(87) Numéro de publication internationale: WO9308844

(56) Documents cités:
- EP-A- 0 273 782
- EP-A- 0 334 301
- EP-A- 0 336 822
- EP-A- 0 415 731
- WO-A-85/05636
- WO-A-90/11359
- WO-A-90/12087
- WO-A-91/02797
- WO-A-92/15693
- PROCEEDINGS OF THE NATIONAL ACADEMY OF SCIENCES OF USA. vol. 87, no. 22, Novembre 1990, WASHINGTON US pages 8746 - 8750 L.K. VENKATESH ET AL. 'Selective induction of toxicity to human cells expressing human immunodeficiency virus type 1 Tat by a conditionally cytotoxic adenovirus vector' cité dans la demande
- PROCEEDINGS OF THE NATIONAL ACADEMY OF SCIENCES OF USA. vol. 85, no. 20, Octobre 1988, WASHINGTON US pages 7572 - 7576 E. BORRELLI ET AL. 'Targeting of an inducible toxic phenotype in animal cells' cité dans la demande

## Description

L'invention est relative à des médicaments et cellules transformées pour la prévention ou le traitement de maladies induites par des virus, notamment rétrovirus pathogènes ayant un tropisme spécifique pour ces cellules.

Dans l'une de ses premières variantes, l'invention est relative à des cellules piégées à l'égard de ces virus et à des médicaments pour la prévention ou le traitement des maladies induites par ces virus, notamment rétrovirus, plus particulièrement en association avec lesdites cellules piégées.

Lorsque l'on parle de rétrovirus, on pense naturellement de façon immédiate au syndrome d'immunodéficience acquise (SIDA) induit chez l'homme par des rétrovirus du type HIV (VIH). Les maladies induites par HIV résultent de la persistance d'une infection virale qui se propage dans des cellules matures CD4 positives, lymphocytes, monocytes ou cellules dendritiques (Klatzmann et al, 1990). Ces cellules matures sont en général considérées comme dérivées de cellules souches communes de la moelle osseuse. Cette origine commune est à la base de l'approche thérapeutique de lutte contre HIV déjà envisagée par certains auteurs et comprenant l'introduction dans les cellules souches hématopoïétiques d'une information génétique qui permettrait l'inhibition de la réplication et la propagation de HIV, en d'autres termes une "immunisation intracellulaire" contre ces rétrovirus (Baltimore, 1988). Ainsi a-t-on déjà proposé de mettre en oeuvre des mutants de certaines protéines de régulation ou protéines structurales du HIV qui seraient aptes à exercer un effet trans-dominant négatif sur la réplication de HIV (Malin et al, 1989 ; Trono et al, 1989). D'autres approches d'immunisation intracellulaire sont fondées sur des tentatives d'inhibition de l'expression du HIV par des ARNs anti-sens (Sczakiel et al, 1991) ou de séquences compétitives HIV-TAR (Sullenger et al, 1990).

L'éventualité d'une intervention thérapeutique assurant l'élimination des cellules T déjà infectées qui agiraient en tant que réservoirs de rétrovirus à l'origine du développement de l'infection a également été évoquée (Venkatesh et al, 1990). Cette approche était fondée sur la constatation faite par ces derniers auteurs que des cellules humaines HeLa ou Jurkat exprimant de façon constitutive la protéine TAT de HIV et transformée par un adénovirus sélectif modifié par un ADN recombinant contenant une séquence nucléique codant pour une thymidine kinase de l'herpes simplex 1 (HSV1-TK) sous le contrôle d'un LTR de ce rétrovirus, étaient plus facilement détruites en présence d'un analogue de nucléoside, le ganciclovir (GCV, 9-(1,3-10 hydroxy-2-propoxyméthyl) guanine) que les mêmes cellules également transformées par le même adénovirus recombinant, mais dépourvues de séquences codantes pour la protéine TAT de HIV .

Les étapes essentielles de la stratégie, qui représenterait en fait une tentative de transposition de la technique dite "d'oblitération de la thymidine kinase" décrite par Borelli et al, 1988, à l'obtention d'une toxicité conditionnelle dans des cellules humaines exprimant des protéines régulatrices TAT et REV de HIV, n'ont pas été indiquées, il est cependant douteux d'une telle stratégie aboutisse, du moins dans les conditions qui ont été suggérées, à un procédé applicable en clinique, dans la lutte ou la prévention du SIDA.

Mais pour effectivement bloquer la réplication de HIV, toutes ces approches impliquent une expression constitutive de niveau élevé des protéines ou ARNs spécifiquement mis en oeuvre. La nécessité d'atteindre à cette expression élevée, explique peut-être les obstacles rencontrés pour obtenir une inhibition complète de la réplication de HIV à l'occasion des essais de mise en oeuvre de certaines de ces stratégies, dans la masure où certaines d'entre elles ont fait l'objet d'investigations plus étendues.

Dans l'une de ses premières approches, l'invention a pour but de remédier à ces inconvénients, voire même de tirer profit dans certains cas des caractéristiques de développement des virus en cause, notamment des rétrovirus susceptibles de conduire à des syndromes d'immunodéficience acquise chez l'homme ou l'animal. Cette approche est fondée sur la présence, assurée au préalable chez l'hôte, de cellules elles-mêmes non infectées, ais piégées à l'égard des virus, notamment rétrovirus, en cause.

L'invention décrit plus particulièrement une population de cellules, notamment hématopoïétiques, piégées à l'égard d'un virus pathogène déterminé ayant un tropisme spécifique pour ces cellules, caractérisée en ce que ces cellules, non infectées par ce virus, contiennent une séquence génétique dont le produit d'expression normalement faiblement exprimé (expression basale) au sein de ces cellules, peut, lorsque sa production est accrue au delà d'un seuil et en quantité suffisante au sein de ces cellules piégées, réagir in situ avec une substance pharmaceutiquement active pour induire la destruction de ces cellules, et des moyens spécifiquement inductibles par un signal fourni par ce virus pathogène, lorsque ce virus pénètre dans ces cellules, pour déclencher la susdite production accrue.

L'administration d'une telle substance pharmaceutiquement active à un hôte porteur d'une telle population de cellules piégées, peut permettre la protection efficace de l'hôte contre une infection par le virus en cause. Il en sera tout particulièrement ainsi, dès lors qu'une grande proportion, sinon la totalité, des cellules spécifiques correspondantes de l'hôte se trouveront être ainsi piégées. En raison de la destruction des cellules piégées commandée par le virus lui-même, la propagation de ce dernier sera bloquée.

L'invention décrit également l'utilisation pour la production de médicaments actifs contre une infection par des virus déterminés à caractère pathogène et ayant un tropisme spécifique pour des cellules, notamment hématopoïétiques d'une molécule pharmaceutiquement active capable, lorsqu'elle se trouve associée in vivo avec une population de cellules de ce type mais piégées contre ce virus pathogène, d'interagir avec le produit d'expression de la séquence génétique contenue dans ces cellules piégées pour produire la destruction sélective de celles des cellules piégées de l'hôte dans lesquelles le virus pénètre.

Dans une forme de mise en oeuvre préférée, la population de cellules piégées est caractérisée en ce que les moyens permettant le déclenchement spécifique de l'accroissement de la production du produit d'expression au delà du seuil susdit et la séquence génétique susdite sont contenus dans une séquence d'ADN recombinant contenu dans ces cellules. Avantageusement, les moyens spécifiquement inductibles par un signal fourni par le virus consistent en un promoteur faiblement actif dans des conditions normales mais susceptible d'être spécifiquement activé lorsque ce virus pénètre dans ces cellules et d'être reconnu par les facteurs de transcription alors présents dans ces cellules. Le produit d'expression de la séquence génétique placée sous le contrôle de ce promoteur peut alors, lorsque sa production est accrue sous l'effet de l'activation du promoteur, réagir avec une substance pharmaceutiquement active, pour former un produit de réaction capable d'induire directement ou indirectement la destruction à la fois des cellules et du virus en cause.

Avantageusement la substance pharmaceutique utilisée induit directement ou indirectement l'interruption in situ de la réplication d'ADNs au sein des cellules on cause. De façon encore particulièrement préférée, l'invention fait également application de la technique dite "d'oblitération de thymidine kinase" déjà décrite par Borelli et al dans des cellules l'exprimant déjà de façon constitutive et dans un champs d'application différent. La population de cellules piégées conformes à un mode de mise on oeuvre préféré de l'invention est alors caractérisée en ce que le produit d'expression codé par ladite séquence génétique placée sous le contrôle dudit promoteur, est une molécule qui, telle la thymidine kinase du virus de l'herpès simplex 1 (HSV1-TK), est apte, lorsqu'elle est présente en une concentration suffisante dans les cellules en cause, à phosphoryler des analogues de nucléosides, tels que l'acyclovir (9-[(2-hydroxyethoxy)méthyl]guanine), en des molécules monophosphatées, elles-mêmes convertibles par des enzymes cellulaires en nucléotides triphosphatés incorporables dans des acides nucléiques en cours d'élongation sous l'effet des polymérases au sein desdites cellules, avec pour effet l'interruption d'élongation de chaînes et la mort cellulaire qui s'ensuit.

Il apparaîtra immédiatement que la "substance pharmaceutique active" utilisable dans un schéma thérapeutique mettant an oeuvre l'une des différentes populations de cellules piégées telles que définies ci-dessus à titre préférentiel doit, dans chaque cas présenter, les propriétés qui lui permettront de réagir avec le produit d'expression correspondant, dans les conditions qui ont été définies.

L'invention trouve une application particulièrement intéressante dans la prévention ou le blocage d'une infection par des rétrovirus infectieux pour l'hôte, en particulier de rétrovirus du type HIV.

A cet égard l'invention décrit donc tout particulièrement des populations de cellules piégées dans lesquelles les cellules en cause sont des monocytes, lymphocytes ou cellules dendritiques CD4 positifs, les moyens spécifiquement inductibles par le virus étant alors avantageusement constitués par tout ou partie des séquences régulatrices propres au rétrovirus en cause et qui contrôlent son expression, notamment par un LTR total ou partiel de ce rétrovirus. Dans le cas de HIV, le moyen d'induction de la production de la substance toxique sera donc naturellement, de préférence constitué par un LTR du HIV concerné. Dans les cellules piégées du genre an question l'ADN recombinant contenu au sein de ces cellules sera normalement dépourvu de toute séquence nucléique autorisant l'expression constitutive ou non de la protéine TAT des rétrovirus concernés.

Dans le cas de HIV, il est avantageux d'avoir recours à un LTR modifié ou partiel autorisant un faible niveau d'expression de la séquence génétique qui lui est associée, niveau qui doit naturellement être très inférieur au seuil, sus-indiqué. Une modification d'un LTR permettant apparemment d'aboutir à un tel résultat dans le cas d'un HIV consiste, par exemple, en une délétion de la région NRE (initiales de "Negative Regulation Element") d'un tel LTR.

A titre d'exemple d'un tel LTR modifié, on citera un LTR de HIV dépourvu de ceux de ses nucléotides situés en aval du nucléotide -167 par rapport au site d'initiation de la transcription (+1), plus particulièrement d'une séquence de LTR s'étendant entre les positions -167 et +80, telle que contenue dans le plasmide pLTR décrit par Sodrosky et al, 1986.

Une autre approche pour s'assurer de l'expression à faible niveau de la séquence génétique associée au LTR consisterait à introduire dans le promoteur un élément de régulation positive destiné à augmenter l'expression basale.

La séquence génétique associée au susdit LTR ou promoteur code de préférence pour une molécule susceptible de n'être rendue toxique (ou dont l'effet toxique ne peut se manifester) qu'à une dose dépassant un certain seuil de concentration, de façon à ce que même une expression spontanée réduite ne mette pas en cause la viabilité des cellules piégées an cause. Tels sont bien en effet les caractéristiques de la protéine HSV1-TK, dont la capacité d'interaction, par exemple avec l'acyclovir, pour le transformer en molécules monophosphatées, est "dose-dépendante".

Les caractéristiques de réplication de HIV font que le principe indiqué ci-dessus lui est applicable avec un avantage particulier, pour obtenir le "suicide" de cellules piégées dès lors que celles-ci seraient présentes dans l'organisme de l'hôte, et cela sous la dépendance même de la pénétration de ce virus dans ces cellules piégées. Tout d'abord le promoteur de HIV est inefficace an l'absence de la protéine régulatrice TAT codée par une séquence génétique correspondante de HIV (Arya et al, 1985 ; Muesing et ai, 1987 ; Cullen, 1990). En outre, le cycle de réplication de HIV implique une première phase dans le temps, d'accumulation d'ARNm. Des ARNm épissés multiples, codant pour des protéines régulatrices de HIV, TAT incluses, sont produites dans cette première phase. Cependant, les ARNm codant pour des protéines de structure de HIV ne sont exprimés de façon significative que 24 heures après l'infection virale. Ce n'est que dans cette deuxième phase que la production de virus s'accélère et devient apparente (Kim et al, 1989).

L'invention met en fait à profit deux phénomènes distincts :
1) l'expression d'un gène toxique placé sous le contrôle d'un promoteur de HIV dans des cellules piégées conformes à l'invention n'est que très faible, tant qu'elles ne sont pas affectées par le rétrovirus ;
2) l'induction de l'expression de la séquence génétique toxique peut être rendue suffisamment rapide dans celle des cellules piégées dans lesquelles le virus pénétrerait, pour déclencher la destruction des cellules en cause et par conséquent du virus lui-même avant même que la cellule soit en mesure de libérer des particules virales nouvellement synthétisées. Par conséquent la propagation virale de tout "virus piégé" est alors bloquée.

L'invention trouve donc une application particulièrement intéressante dans le traitement des infections par des rétrovirus. Elle n'est nullement limitée au traitement des infections par HIV. Elle s'applique également à la prévention ou à l'inhibition de la réplication d'autres rétrovirus, qu'il s'agisse d'autres rétrovirus ayant un tropisme particulier pour les cellules CD4 positives, par exemple des rétrovirus du type HTLV, ou encore des rétrovirus homologues des précédents et infectieux pour des mammifères autres que l'homme.

Pour construire des cellules piégées pour l'homme, conformes à l'invention, on peut avoir recours à toute technique adéquate, notamment par infection in vitro des cellules correspondantes par une pseudo-particule virale de type Moloney amphotrope, qui infecte donc aussi des cellules humaines. Ces particules virales sont produites par une lignée cellulaire dite de "packaging" que l'on aura construite au préalable. Une lignée de packaging est capable de fabriquer tous les éléments structuraux constituant une particule virale, mais est incapable d'introduire dans des particules virales en voies de maturation les ARNs viraux produits par cette lignée cellulaire. C'est pourquoi, ces lignées dites de packaging fabriquent en permanence des particules virales vides. L'introduction d'une construction génétique appropriée, qui contient l'ADN recombinant tel qu'il a été défini plus haut, permet à ces lignées de packaging d'être introduites dans les particules virales vides réalisant ainsi des pseudo-particules virales. Ces pseudo-particules virales sont capables d'infecter différentes cellules cibles, cellules cibles qui varient selon la lignée de packaging qu'on a utilisée au départ. Par exemple, si cette lignée de packaging est dérivée d'un virus dit de Moloney amphotrope, les particules virales produites infectent parfaitement les cellules hématopoïétiques humaines.

Ainsi les cellules à piéger, notamment des cellules hématopoïétiques, sont-elles mises en présence d'un surnageant de culture provenant de la lignée de packaging qui produit les pseudo-particules virales amphotropes. Les cellules à piéger sont par exemple co-cultivées directement avec les cellules de packaging. Pendant cette étape de co-culture ou de culture, les particules amphotropes présentes dans la suspension infectent les cellules à piéger et introduisent donc dans celles-ci les éléments génétiques qu'elles contiennent.

Pour la production de ces cellules piégées mettant plus particulièrement en oeuvre un LTR de rétrovirus, notamment de HIV, on aura avantageusement recours aux vecteurs rétroviraux murins amphotropes et aux lignées cellulaires de packaging (respectivement telles qu'elles ont été revues par Weatherall, 1991 et Friedman, 1989). Les techniques pour la production de lignées de cellules transformées par un tel vecteur rétroviral (voir par exemple Danos et al, 1988 et Markowitz et al, 1989), peuvent être transposées à la production de cellules piégées, notamment hématopoïétiques conformes à l'invention. De même les techniques de transfert génétique mettant en oeuvre de tels systèmes (Kasid et al, 1990) peuvent-elles être appliquées au transfert, chez l'homme, des cellules piégées telles qu'elles ont été définies plus haut.

Une population intéressante de cellules piégées conformes à l'invention est dérivée de cellules de moelle osseuse immatures, provenant d'un prélèvement de moelle osseuse, et dépourvues des marqueurs de surface caractérisant des cellules hématopoïétiques matures, et préalablement transformées en "cellules piégées" dans les conditions sus-indiquées. Elles sont particulièrement adaptées à la mise en oeuvre de l'un des protocoles thérapeutiques dont les étapes sont brièvement rappelées ci-après.

Les étapes essentielles de ce protocole sont alors les suivantes :
- prélèvement de moelle osseuse chez le patient, et traitement de cette moelle osseuse selon les techniques de l'art, de façon à purifier les cellules souches immatures dépourvues des marqueurs de surface caractérisant les cellules hématopoïétiques matures ;
- incorporation dans ces cellules immatures du susdit ADN recombinant contenant le promoteur activable par le virus pathogène et, placé sous le contrôle de ce promoteur, la séquence génétique codant par exemple pour HSVI-TK dont le produit d'expression peut interagir avec la substance pharmaceutiquement active administrée ultérieurement, en l'occurrence des analogues appropriés de nucléosides du type acyclovir ou gancyclovir, pour bloquer la réplication cellulaire dans les cellules dans lesquelles pénétrerait le virus pathogène ;
- Les cellules de la moelle osseuse obtenue, qui contient alors l'ADN recombinant défini plus haut, sont soit réinjectées en totalité chez le patient, soit soumises à une étape de culture supplémentaire et de sélection pour obtenir une quantité de cellules ayant effectivement été infectées par les particules amphotropes et donc contenant et exprimant l'information génétique contenue dans les particules pseudo-virales. Les cellules de moelle osseuse manipulées sont réinjectées par voie intraveineuse au patient qui aura été préalablement conditionné, par exemple par traitement avec de fortes concentrations d'antiviraux appropriés, utilisés à des doses fortes même si celles-ci sont relativement toxiques pour la moelle osseuse puisque le patient recevra par la suite de nouvelles cellules de moelle osseuse. Ce conditionnement préalable du patient peut même impliquer une destruction in vivo de son système immunitaire initial, notamment par irradiation. Dans cette dernière hypothèse, les cellules piégées réinjectées chez le patient -ou les cellules piégées provenant d'une moelle osseuse issue d'un donneur différent, traitées comme indiquées ci-dessus et injectées au patient- sont alors aptes à se renouveler constamment chez l'homme et à donner naissance aux différentes catégories de cellules hématopoïétiques qui, alors, portent toutes la séquence recombinante ;
- administration de la substance pharmaceutiquement active au patient pendant le temps requis, notamment pour entraîner la destruction sélective des cellules piégées, lorsque celles-ci sont infectées par le virus pathogène, suite à l'interaction de la substance pharmaceutiquement active avec le produit d'expression, notamment HSVI-TK alors produit in situ en concentration suffisante dans ces cellules piégées sous l'effet de l'activation du promoteur, notamment LTR lorsque le virus pathogène est un rétrovirus.

Naturellement les modalités de l'administration de la substance pharmaceutiquement active, notamment l'acyclovir ou le gancyclovir, doivent être mises au point par la clinicien. Elles comportent, par exemple, un traitement continu dans un premier temps, itératif dans un second temps.

Mais l'invention ne saurait, au niveau de son application thérapeutique, être limitée au cas précédemment évoqué. Le prélèvement de moelle osseuse peut, dans certains cas, être remplacé par un simple prélèvement sanguin. Dans ce cas ce ne sont pas les cellules souches qui sont manipulées, mais les lymphocytes matures. Ceci simplifie la procédure d'obtention des cellules à manipuler, mais oblige à reproduire le traitement de façon itérative compte tenu de la courte durée de vie des lymphocytes matures.

Cette procédure peut être néanmoins intéressante dans le cas de l'infection par le HIV. On peut réinjecter de façon répétée à un patient séropositif, soit ses propres lymphocytes, soit les lymphocytes d'un donneur HLA compatible, qui auraient été manipulés de la façon décrite auparavant. Des populations hybrides de cellules CD4, piégées contre une infection par la HIV, coexisteront alors chez le patient avec des populations de cellules CD4 positives sensibles. Le résultat serait quand même certainement une réduction de la capacité de dissémination du virus dans l'organisme, ce qui peut dans certains cas être suffisant pour permettre au système immunitaire de l'hôte de surmonter l'infection et d'éliminer totalement le virus.

Dans cet esprit les étapes du protocole thérapeutique seraient les suivantes :
- prélèvement des cellules mononucléées du sang périphérique ;
- culture des lymphocytes ainsi obtenus avec les cellules produisant les particules virales amphotropes ;
- sélection éventuelle des cellules exprimant le transgène de la séquence recombinante et mise en culture de ces cellules. Ces cellules sont, le cas échéant, préalablement mises en expansion selon des procédures classiques, notamment après activation des cellules en présence d'interleukine-2 et mises en culture, ce qui permet une expansion extrêmement importante du nombre des lymphocytes ;
- réinjection des cellules aux patients par voie intraveineuse.

Quelle que soit la procédure mise en oeuvre, l'activation du LTR dans les lymphocytes portant la séquence recombinante entraîne alors le déclenchement de l'expression des gènes codant pour la substance toxique ; la destruction des cellules infectées interrompt alors le processus de propagation virale qui normalement conduit au SIDA.

Il va de soi que dans les cas où l'on ne procéderait pas à une substitution totale de la moelle osseuse initiale du receveur par une moelle osseuse traitée, il conviendra de s'assurer de l'existence chez le receveur d'une proportion adéquate de cellules piégées vis-à-vis des cellules sensibles non piégées, de façon à ce que le système immunitaire de l'hôte soit effectivement mis en condition de surmonter l'infection virale, comme cela a été indiquée plus haut.

Il peut aussi être envisagé d'administrer aux patients des rétrovirus défectifs qui pénétreraient dans une partie de leurs lymphocytes CD4 sans s'y développer. L'infection ultérieure des mêmes cellules par le rétrovirus infectieux entraînerait alors la destruction des cellules en question et un ralentissement éventuellement suffisant de la dissémination virale.

Conformément à la présente invention, les cellules modifiées dans les conditions sus-indiquées peuvent être mises en oeuvre dans un traitement --et plus particulièrement dans le cas de leur utilisation ultérieure dans le cadre de la prévention ou d'un traitement du SIDA- en association avec l'AZT (abréviation de azido-thymidine).

De nos jours, l'AZT est le médicament le plus couramment utilisé chez les personnes séropositives et les malades atteints de SIDA, mais les doses utilisées en thérapeutique engendrent des effets secondaires importants, et le problème majeur est une perte de l'activité thérapeutique liée à l'apparition de variants du VIH résistant à l'AZT. Cette drogue est phosphorylée par la thymidine kinase cellulaire en AZT-monophosphate qui va être ensuite transformée en AZT-diphosphate et AZT-triphosphate par d'autres enzymes cellulaires. Il est présumé que la forme triphosphate de la molécule est celle qui inhibe la réplication virale lors de la reverse transcription.

L'invention repose sur la découverte que l'action de l'AZT introduit dans des cellules de l'hôte le recevant devait être potentialisée par un accroissement de la quantité ou de la qualité de thymidine kinase produite dans au moins certaines de ces cellules. Tel est bien le résultat susceptible d'être obtenu, à l'occasion de la réinjection chez l'homme des susdites cellules, notamment de la moelle osseuse et notamment par mise en oeuvre d'un protocole thérapeutique tel que décrit ci-dessus, mais dans lequel la substance pharmaceutiquement active consisterait en AZT, en lieu et place de l'acyclovir, le gancyclovir ou substance analogue.

Ainsi a-t-il été montré, qu'après introduction dans la lignée lymphocytaire HUT-78 d'une séquence codant pour la thymidine kinase de l'Herpès HSV-1 (HUT-TK) sous le contrôle des séquences régulatrices du VIH qu'il était possible, après infection de ces cellules par le VIH, de bloquer la dissémination virale en présence de doses d'AZT plus faibles que celles nécessaires à l'obtention du même résultat dans les cellules des mêmes lignées lymphocytaires, ne contenant cependant pas la susdite séquence codante.

La lignée HUT-TK possède en effet deux thymidines kinase : sa thymidine kinase cellulaire et la thymidine kinase de l'Herpès. L'apport de cette deuxième thymidine kinase augmente de façon importante la formation d'AZT triphosphate. En effet, les cellules HUT-TK et la lignée parentale incubées en présence d'AZT radiomarqué et la purification des différentes formes phosphorylées a montré une production de la forme triphosphate trois fois plus importante dans la lignée HUT-TK que dans la lignée parentale. Il a aussi été montré qu'il est possible de protéger la lignée HUT-TK avec une dose d'AZT 3 à 10 fois moins importante que pour la lignée HUT-78.

En conclusion, l'apport du gène de la thymidine kinase de l'Herpès permet donc d'augmenter de façon importante les propriétés antivirales de l'AZT vis-à-vis de l'infection par le VIH.

En conséquence de quoi l'invention concerne aussi l'utilisation pour la production de médicaments actifs contre une infection par des rétrovirus HIV de l'AZT ou d'un analogue nucléosidique thérapeutiquement actif de l'AZT, tel que la dideoxycytidine (DDC) ou la dideoxynosine (DDI), associé in vivo à une population de cellules hématopoïétiques contenant une séquence génétique hétérologue codant pour une thymidine kinase, notamment une thymidine kinase du virus de l'Herpès simples 1 (HSV-TK) sous le contrôle d'un promoteur assurant une expression basale non toxique pour l'hôte.

Ce promoteur est le cas échéant constitué par un LTR de HIV, notamment un LTR partiel peu modifié comme cela a été indiqué ci-dessus.

La présence du transgène dans certaines des cellules, notamment lorsque celles-ci ont été administrées selon un protocole semblable à celui qui a été décrit plus haut, a donc pour objectifs l'accroissement de l'efficacité de l'AZT qui peut également être administré en quantités ou concentrations moins importantes, et la prévention de l'apparition de mutants résistants à l'AZT. Il est probable que l'action concertée de l'AZT et de l'acyclovir ait des effets additifs, voire synergiques. En effet, leurs modes d'actions sont situés à des étapes distinctes du cycle réplicatif du VIH. D'ailleurs cette dernière propriété devrait prévenir l'apparition de mutants résistants à l'AZT.

On appréciera aisément que les deux variantes de l'invention qui viennent d'être décrites peuvent en fait être appliquées de façon interchangeable ou séquentielle dans un protocole clinique susceptible d'intéresser le même patient. Ainsi, peut-on faire alterner chez un patient dont une partie au moins des cellules contiendrait le transgène, des administrations d'acyclovir ou analogue, dans le but de promouvoir le "suicide" de cellules infectées par le rétrovirus et (pour le cas où une éradication complète des cellules infectées n'aurait été produite) des administrations d'AZT alors rendues beaucoup plus efficaces. Ces traitements alternés peuvent être envisagés de façon à présenter un caractère cyclique. L'invention met ainsi à la disposition du clinicien des possibilités importantes de modulation du traitement clinique, selon les degrés d'atteinte ou la phase du développement de la maladie chez l'hôte concerné.

L'invention concerne donc également une association à des fins thérapeutiques :
- de cellules hématopoïétiques contenant une séquence génétique hétérologue codant pour une thymidine kinase sous le contrôle d'un promoteur assurant une expression basale non toxique pour l'hôte,
- d'un médicament consistant en une molécule pharmaceutiquement active capable, lorsqu'elle se trouve associée in vivo à la susdite population de cellules, à interagir avec le produit de la séquence génétique codant pour la thymidine kinase hétérologue,
- de l'AZT ou d'un analogue nucléosidique thérapeutiquement actif de l'AZT, tel que la dideoxycytidine (DDC) ou la dideoxynosine (DDI).

Mais il apparaîtra également à l'homme du métier que la deuxième variante (mettant en oeuvre l'AZT) peut également être utilisée indépendamment de la première variante. Dans cette dernière hypothèse, et compte tenu du caractère peu toxique des thymidines kinase susceptibles d'être employées. il doit être remarqué que les contraintes pesant sur le promoteur sous le contrôle duquel la séquence génétique en question se trouve alors placée sont moindres que dans le cas de la première variante de l'invention. En particulier, le transgène pourrait également être constitué par un ADN recombinant contenant la séquence nucléique codant pour la thymidine kinase, notamment HSV1-TK sous le contrôle d'un promoteur éventuellement plus fort.

Mais on appréciera que si ce promoteur plus fort est de nature à contribuer à la plus forte potentialisation de l'efficacité de l'AZT administré au patient, il pourrait ne plus autoriser l'administration à titre préventif de l'acyclovir, chaque fois que l'on voudra bénéficier de l'"effet suicide" susceptible d'être induit par l'acyclovir à l'occasion de l'infection des cellules concernées par VIH.

Les résultats expérimentaux qui suivent n'ont d'autre but que celui d'illustrer l'efficacité des interactions entre des cellules piégées conformes à l'invention avec des substances pharmaceutiquement actives correspondantes dans un système expérimental mettant en jeu un HIV. Il sera fait référence aux dessins, dans lesquels :

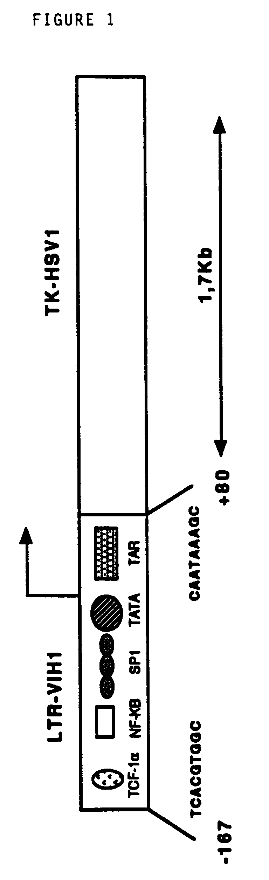

- la **figure 1** montre de façon schématique la structure d'une cassette vecteur contenant des séquences régulatrices du VIH-1 et, sous leur contrôle, un gène de la thymidine kinase du virus Herpès simplex de type 1 ;

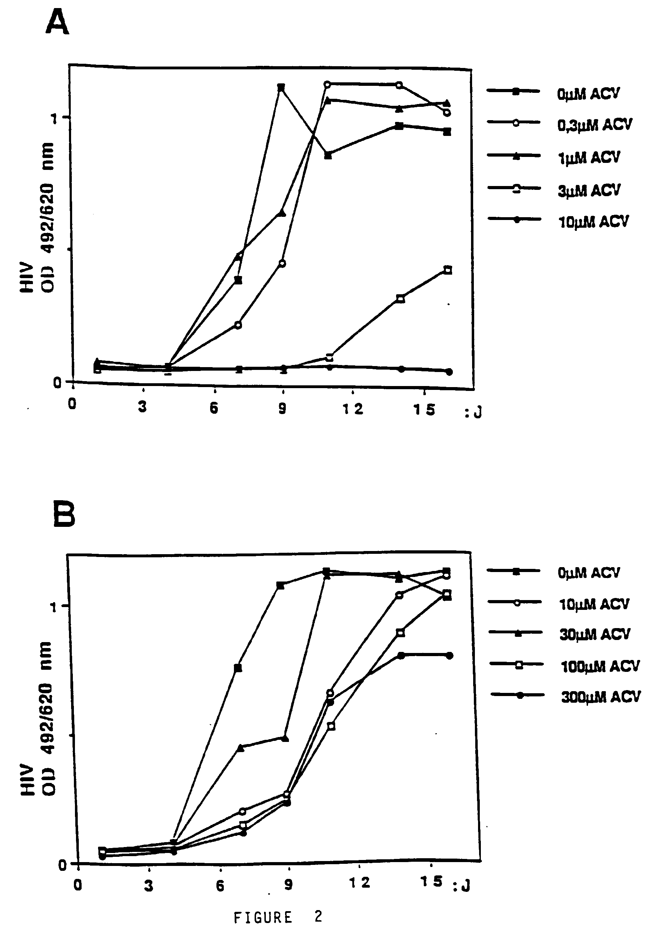

- les **figures 2A** et **2B** fournissent des courbes illustrant les effets de différents concentrations en Acv sur la réplication de HIV dans des cellules respectivement piégées et non piégées, cette réplication étant appréciée par les taux d'antigènes de HIV mesurés dans les surnageants de cellules étudiées, exprimés par des mesures de densité optique (OD à 492/620 nm sur les axes des ordonnées) en fonction de la durée des essais (en jours J sur l'axe des abscisses) ;

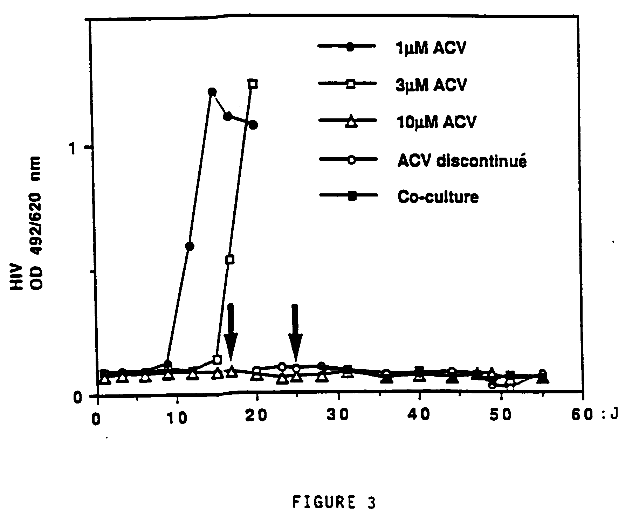

- la **figure 3** témoigne de l'effet de différentes concentrations d'Acv sur le degré de réplication cellulaire de HIV dans les cellules piégées, ce degré étant apprécié comme dans le cas des figures **2A** et **2B**, en fonction de la durée des essais, en jours, et les prolongements de ces effets après séparation de l'Acv, et à l'occasion de nouvelles tentatives d'infections rétrovirales ;

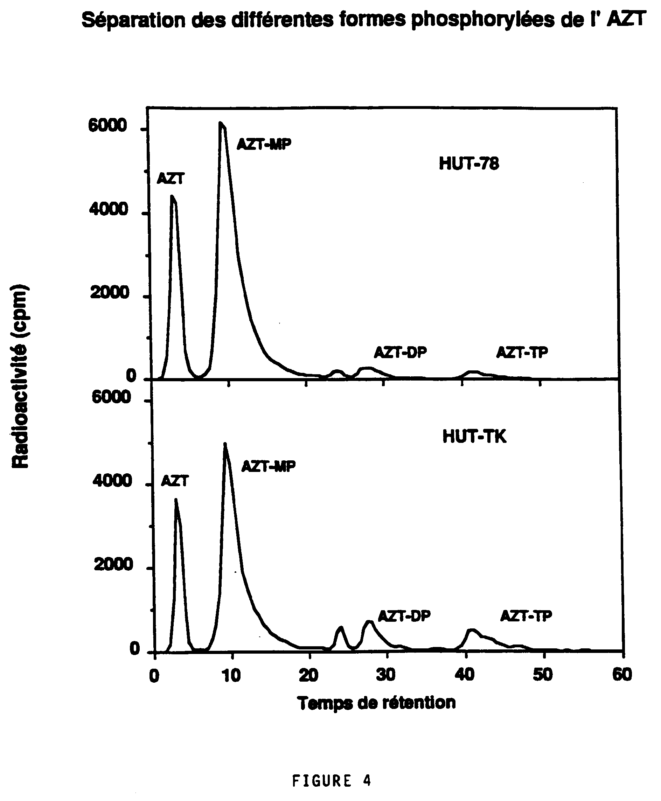

- les **figures 4A** et **4B** montrent les résultats comparés des mesures des temps de rétention en FPLC (Fast Purification Liquid Chromatography) des différentes formes phosphorylées de l'AZT incorporées dans des cellules HUT-78 (fig. **4A**) et HUT-TK respectivement dans un protocole d'essai ayant compris leur mise en contact avec de l'AZT marqué par du tritium ;

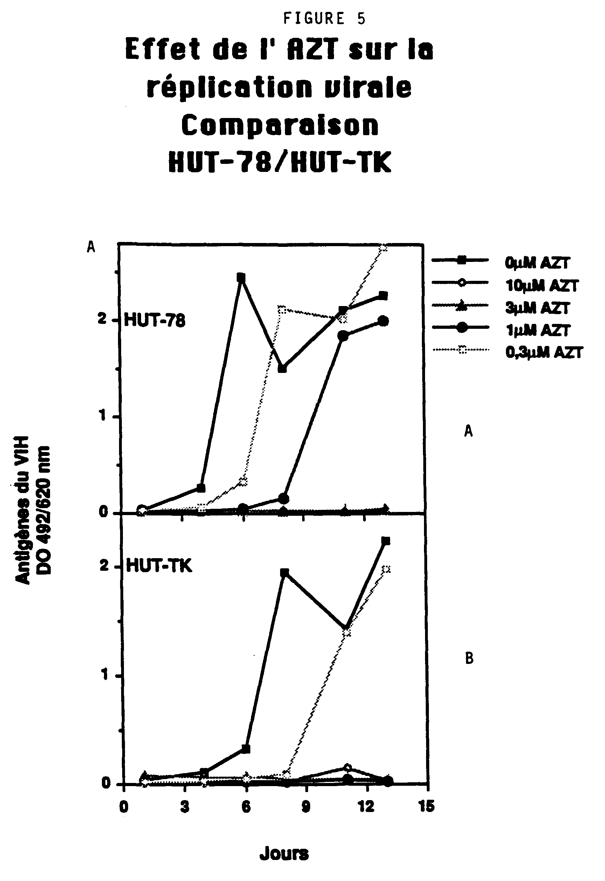

- les **figures 5A** et **5B** montrent à titre comparatif les efficacités des effets d'inhibition par les mêmes doses d'AZT à l'égard de la réplication de HIV-1 dans des cellules HUT-78 et HUT-TK respectivement préalablement infectées par le rétrovirus.

### Expression dans des cellules lymphoïdes CD4+ d'un gène HSV-TK placé sous le contrôle d'un LTR de HIV

On décrit d'abord la construction d'un plasmide (schématisé dans la figure **1**) dans lequel le gène HSV1-TK a été placé sous le contrôle d'un 5'-LTR de HIV-1 (pLTR-TK). L'utilisation de ce plasmide pour la transformation de cellules lymphoïdes CD4+ est ensuite décrite.

### Construction du plasmide

Le plasmide pLTR a été obtenu à partir du plasmide pIII env(3-1) (Sodrosky et al, 1986). La séquence Sal1/Xba1 env a été délétée de ce plasmide. Les extrémités du plasmide ont été rendues franches en présence d'une enzyme de Klenow puis recircularisées par ligation. Pour fabriquer le plasmide pLTR-TK, l'insérat BglII/BamHI de 2,7 Kb contenant la séquence HSV1-TK issue du plasmide pFG5(Colbere-Garapin et al, 1978) a été cloné dans le site BamH1 du plasmide pGEM3Z (Promega). L'insérat contenant le gène HSV1-TK et délimité par des extrémités HindIII et PvuII obtenu à partir de ce plasmide, a ensuite été cloné dans des sites de clonage HindIII/SmaI du plasmide pLTR. C'est ainsi qu'à été obtenu le plasmide pLTR-TK. Afin de faciliter l'obtention de transformants stables, le fragment XhoI/BamHI de 2,8 Kb obtenu à partir de pLTR a été fusionné avec un fragment SalI/BamHI obtenu à partir du plasmide pHYG (Sugden et al, 1985) qui code pour une résistance à l'hygromycine.

Toutes ces opérations de clonage ont été effectuées en utilisant des techniques de clonage classique (Maniatis et al, 1982).

### Transfection d'ADN dans des cultures cellulaires

Le plasmide pLTR-TK a été introduit dans des cellules lymphoïdes HUT-78 CD4+ par electroporation et des transformants stables ont été sélectionnés avec de l'hygromycine. A cette fin, les cellules HUT-78 ont été maintenues dans un milieu RPMI additionné de sérum de veau foetal à 10 % (Flow). Pour la transfection, les cellules ont été rincées avec du PBS glacé, sédimentées par centrifugation, rincées à nouveau puis resuspendues dans le milieu RPMI à une densité de 4 millions de cellules/ml. Elles ont ensuite été maintenues dans de la glace pendant 10 mn. La quantité de 0.8 ml de cette suspension cellulaire a ensuite été ajoutée dans une cuvette refroidie d'electroporation en mélange avec 20 µg du plasmide. Deux impulsions électriques consécutives (2,5 KV ; 0,25 µF et 0,3 KV ; 960 µF) ont ensuite été appliquées aux cellules en utilisant un électroporateur BIORAD. Les cellules ont été mises au repos sur de la glace pendant 10 mn, puis remises en suspension dans 50 ml du milieu de culture avant d'être distribuées dans les 48 heures, dans les puits d'une plaque MULTIWELL (Falcon). 48 heures plus tard 500 µg/ml d'hygromycine ont été ajoutés. La moitié du volume du milieu a été éliminée de façon douce sur une durée de 3-4 jours. Les cellules ont ensuite été cultivées dans le milieu de sélection jusqu'à l'apparition de clones.

Six clones transformés (ci-après dénommés HVT-TK) ont été obtenus et analysés par Southern Blot. 15 µg d'ADN génomique digéré avec EcoR1 ont été hybridés avec un insérat TK marqué au ³²P obtenu à partir d'un produit de digestion de pLTR-TK avec l'enzyme HindIII.

Cette analyse a révélée la présence d'une copie du gène HSV1-TK par cellule transformée. En revanche aucune hybridation n'a pu être détectée entre l'ADN génomique des cellules parentales HUT-78 (non transformées) avec la même sonde. De même aucun signal traduisant la présence d'ARN messagers de HSV1-TK n'a été détecté dans les cellules de HUT-78.

Dans 10 essais indépendants d'infection par HIV, la cinétique de la réplication du rétrovirus, la quantité des antigènes de HIV produites en culture et les effets cytopathiques du virus se sont avérés semblables dans les cellules transformées (HUT-TK) et dans les cellules parentales non transformées (HUT-78).

### Examen de la sensibilité des lignées HUT-78 et HUT-TK à l'acyclovir (Acv)

Le taux de réplication de l'ADN a été apprécié par le taux d'incorporation de thymidine tritiée dans les cellules testées et par la mesure de la viabilité des cellules dans le test d'exclusion au bleu de trypan.

A cet effet les cellules HUT-78 et HUT-TK ont été cultivées on présence de concentration croissante d'Acv. Après 6 jours de culture, les effets de l'Acv sur la réplication d'ADN et de survie cellulaire ont été appréciés par incorporation de thymidine tritiée et par le comptage direct on présence de bleu de trypan respectivement.

Il résulte de l'examen des résultats que les cellules HUT-TK témoignent certes d'une sensibilité plus élevée que celle des cellules parentales non transformées. La concentration d'Acv inhibitrice 50 % (ID50) s'est avérée être d'environ 30 µM, au lieu de 300 µM pour les cellules parentales.

La plus grande sensibilité des cellules HUT-TK à l'Acv indique que ces cellules, qui expriment l'ARNm de HSV1-TK, synthétisent un HSV1-TK fonctionnel. Mais cet accroissement de sensibilité de HUT-TK est modéré et aucune mort cellulaire n'a été détectée lorsque ces cellules sont cultivées en présence de 10 µM de Acv, c'est à dire à une concentration à laquelle les cellules HUT-TK peuvent être maintenues en culture indéfiniment.

Le faible taux basal d'expression de HSV1-TK dans les cellules HUT-TK qui est en revanche considérablement accru en présence de la protéine TAT produite à l'occasion de la réplication de HIV, alors que dans des conditions semblables les cellules HUT-78 ne sont que peu affectées.

### Effet de l'Acv sur une infection par HIV de cellules parentales et de cellules exprimant le gène HSV1-TK (HUT-TK) et de cellules parentales (HUT-78)

Les cellules ont été infectées par HIV pendant 3 heures, puis cultivées an présence de Acv, utilisé an des concentrations s'étageant de 0,3 à 10 µM pour les cellules HUT-TK et de 10 à 300 µM pour les cellules HUT-78.

Comme le montre la figure **2A**, on a constaté des cinétiques de réplication de HIV semblables dans des cellules HUT-TK cultivées an présence de doses d'Acv correspondant respectivement à 0,3 et 1 µM d'Acv. La première apparition d'antigènes de HIV a été noté au 7ème jour. A la dose de 3 µM d'Acv, la première apparition d'Acv dans les cellules HUT-TK a au contraire été différée pendant une semaine entière. Mais à la concentration an Acv de 10 µM l'infection par HIV a été totalement inhibée pendant les 17 jours de durée de la culture . Des résultats identiques ont été obtenus à l'issue de trois expériences indépendantes.

En revanche la réplication de HIV a été détectée au jour 7 dans toutes les cellules HUT-78, et ce pour toutes les concentrations de Acv (figure **2B**). A la dose de 10 µM an Acv, aucune modification significative de la réplication de HIV n'a pu être observée. A une concentration de 300 µM en Acv, on n'observe qu'une faible décroissance de la quantité d'antigènes HIV détectés dans la culture.

### Effet à long terme de l'Acv sur l'infection par HIV de cellules exprimant HSV1-TK

Le caractère "définitif" de l'inhibition de la réplication de HIV dans les cellules HUT-TK an présence de la dose de 10 µM an Acv a été apprécié dans l'essai suivant.

Des cellules de HUT-TK ont de nouveau été cultivées an présence de 10 µM d'Acv. Comme dans l'essai précédent, les cellules HUT-TK se sont avérées être complètement protégées vis-à-vis de la réplication de HIV pendant une phase de culture initiale de 17 jours.

A ce point la culture a été divisée en deux parties. La première partie de la culture a été maintenue en présence de 10 µM de Acv tandis que la deuxième partie a été cultivée en l'absence de Acv. Au jour 25, les cellules qui avaient été maintenues en présence de la concentration de 10 µM en Acv ont de nouveau été divisées en deux cultures. L'une d'entre elles a ensuite été co-cultivée avec une culture parentale de cellules HUT-78.

Aucune réplication n'a été détectée dans aucune desdites cultures pendant une durée de 2 mois (figure **3**). Il est remarquable que les cellules HUT-TK, initialement infectées avec HIV et cultivées en présence de 10 µM de Acv, se soient avérées cultivables de façon indéfinie sans aucun effet toxique. Pris dans leur ensemble, ces résultats indiquent que les cellules de HUT-TK peuvent, en tant que population, être totalement protégées de la réplication par HIV.

L'absence de détection dans les cultures HUT-TK traitées par Acv, confirme également que l'expression de HSV1-TK est déclenchée de façon très précoce à l'occasion du cycle de réplication de HIV, et ce avant môme que des protéines de structure soient synthétisées et des particules virales libérées dans le milieu de culture. Le "suicide" des cellules individuelles HUT-TK infectées par le virus et ce, dès la première phase de la réplication virale, à conduit à une protection de toute la population des cellules des cultures en cause.

De ces résultats découle la présomption sérieuse que la présence chez le patient de proportions substantielles de cellules piégées contre HIV peut conduire à une protection complète chez le patient de l'ensemble de ses cellules CD4+. Pour l'apprécier, il faut se placer dans le contexte de l'infection par HIV qui ne conduit en fait à une maladie chronique, qu'en raison de la constance de la propagation virale d'une cellule CD4+ à l'autre. La présence chez ce patient d'une population de cellules hébergeant le transgène LTR-TK peut donc conduire au blocage, à tout le moins à un freinage de cette propagation virale à un point tel que les défenses immunitaires de l'hôte retrouvent leur capacité à juguler l'infection résiduelle par HIV.

L'invention, ne saurait être limitée aux modes de réalisation qui ont été plus particulièrement illustrés dans les exemples, en particulier ceux mettant en oeuvre le couple d'éléments respectivement formés par la séquence génétique codant pour la HSV1-TK et la "substance pharmaceutiquement active" constituée par l'acyclovir ou un analogue de ce nucléoside modifié.

L'homme du métier est à même d'imaginer d'autres "couples" de réactifs qui peuvent être mis en oeuvre aux mêmes fins. On en citera un, seulement à titre d'exemple, ce couple comprenant alors :
- une séquence génétique codant pour une protéase déterminée capable de cliver une protéine "inerte" de fusion résultant de la réunion d'une toxine, telle qu'une sous-unité toxique de la toxine diphtérique, et d'une autre protéine par l'intermédiaire d'une séquence en acides aminés constituant un site spécifiquement clivable par cette protéase déterminée, dans la mesure où cette protéase serait exprimée in situ en une concentration dépassant un seuil déterminé ;
- une "substance pharmaceutiquement active" constituée par ladite protéine de fusion "inerte".

L'activation des cellules piégées contenant une telle séquence génétique, placée par exemple sous le contrôle d'un LTR de rétrovirus, notamment à l'occasion de la pénétration de ce rétrovirus dans la cellule piégée, entraîne par conséquent la production de la protéase déterminée et, en présence de la protéine de fusion introduite dans les cellules via son administration à l'hôte, un clivage in situ de cette protéine de fusion et une libération de toxine toxique alors apte à sélectivement détruire la cellule et son contenu viral.

### Examen des différente formes phosphorylées de l'AZT

Des cellules HUT-78 et HUT-TK ont été cultivées en présente d'AZT tritié. Après culture, les cellules ont été récupérées, les parois cellulaires rompues et les hydrolysats obtenus soumis à une chromatographie liquide sous haute pression (HPLC).

Les figures **4A** et **4B** montrent les pics respectivement obtenus à partir des cellules HUT-78 et HUT-TK, d'AZT, d'AZT-monophosphorylé (AZT-MP), d'AZT-diphosphorylé (AZT-DP) et d'AZT triphosphorylé (AZT-TP). Il découle de l'examen des graphiques que les proportions d'AZT-DP et d'AZT-TP produites dans les cellules contenant le transgène TK étaient nettement plus élevées que dans les cellules HUT-78 non transformées.

### Examen de la sensibilité des lignées HUT-78 et HUT-TK à l'AZT

Le taux de réplication du HIV a été apprécié par la mesure quantitative des gènes viraux dans le surnageant.

A cet effet les cellules HUT-78 et HUT-TK ont été cultivées en présence des différentes concentrations d'AZT indiquées dans les figures **5A** et **5B** respectivement et infectées par HIV. Les effets de l'AZT sur la réplication de HIV ont été appréciés par dosage des antigènes de HIV.

Il résulte de l'examen des résultats que les cellules HUT-TK témoignent certes d'une résistance à l'infection plus élevée que celle des cellules parentales non transformées. A une dose de 1µM d'AZT, l'infection est jugulée dans les cellules HUT-TK, alors qu'à la même dose l'infection démarre après le 6^{ème} jour et s'accélère dès le 8^{ème} jour.

Dans ce qui précède, il a essentiellement été fait référence à l'utilisation de l'AZT. Mais il va de soi que l'AZT peut être remplacé par tout analogue nucléosidique thérapeutiquement actif de l'AZT, tel que la dideoxycytidine (DDC) ou la dideoxynosine (DDI).

### REFERENCES

Arya, S. K., Guo, C., Josephs, S. F., Wong-Staal, F. (1985). Trans-activator gene of human T-lymphotropic virus type III (HTLV-III). Science 229, 69-73.
Baltimore, D. (1988). Intracellular immunization. Nature 335, 395-396.
Borrelli, E., Heyman, R., Hsi, M., and Evans, R. M. (1988). Targeting of an inducible toxic phenotype in animal cells. Proc. Natl. Acad. Sci. USA 85, 7572-7576.
Colbere-Garapin, F., Chousterman, S., Horodniceanu, F., Kourilsky, P., and Garapin, A. C. (1979). Cloning of the active thymidine kinase gene of herpes simplex virus type 1. in Escherichia coli K-12. Proc. Natl. Acad. Sci. USA 76, 3755-3759.
Cullen, B. R. (1990). The HIV-1 tat protein : an RNA sequence-specific processivity factor. Cell 63, 655-657.
Danos, O., and Mulligan R. C. (1988). Safe and efficient generation of recombinant retroviruses with amphotropic and ecotropic host ranges. Proc. Natl. Acad. Sci. USA 85, 6460-6464.
Elion, G. B. (1983). The biochemistry and mechanism of action of acyclovir. Journal of Antimicrobial Chemotherapy 12, 9-17.
Field, A. K., Davies, M. E., DeWitt, C., Perry, H. C., Liou, R., Germershausen, J., Karkas, J. D., Ashton, W. T., Johnston, D. B. R., and Tolman R. L. (1983). 9-[2-Hydroxy-1(hydroxymethyl) ethoxy]methyl guanine : a selective inhibitor of herpes group virus replication. Proc. Natl. Acad. Sci. USA 80, 4139-4143.
Fyfe, J. A., Keller, P. M., Furman, P. A., Miller, R. L., and Elion, G. B. (1978). Thymidine kinase from herpes simplex virus phosphorylates the new antiviral coumpound, 9-(2-hydroxyethoxymethyl) guanine. Journal of Biological Chemistry 253, 8721-8727.
Kasid, A., Morecki, S., Aebersold, P., Cornetta, K., Culver, K., Freeman, S., Director, E., Lotze, M. T., Blaese, R. M., Anderson, W. F., and Rosenberg, S. A. (1990). Human gene transfer : characterization of human tumor-infiltrating lymphocytes as vehicles for retroviral-mediated gene transfer in man. Proc. Natl. Acad. Sci. USA 87, 473-477.
Kim, S., Byrn, R., Groopman, J., and Baltimore, D. (1989). Temporal aspects of DNA and RNA synthesis during human immunodeficiency virus infection : evidence for differential gene expression. Journal of Virology 63, 3708-3713.
Klatzmann, D. R., McDougal, J. S., Maddon, P. J. (1990). The CD4 molecule and HIV infection. Immunodeficiency Reviews 2, 43-66.
Malim, M. H., Böhnlein, S., Hauber, J., and Cullen, B. R. (1989). Functional dissection of the HIV-1 rev trans-activator - derivation of a trans-dominant repressor of rev function. Cell 58, 205-214.
Maniatis, T., Fritsch, W., and Sambrook, J. (1982). Molecular cloning : a laboratory manual (Cold Spring Harbor, New York : Cold Spring Harbor Laboratory).
Markowitz, D., Goff, S., Bank, A. (1988). A safe packaging line for gene transfer : separating viral genes on two different plasmids. J. Virol. 62, 1120-1124.
McCune, J. M., Namikawa, R., Lieberman, M., Weissman, I., and Kaneshima, H. (1990). The SCID-hu mouse as a model system for HIV infection. In Human Retroviruses, J. E., Groopman, I. S., Chen, S. Y., Essex, M., and Weiss, R. A., eds. (New York : Liss, A. R., Inc.), pp. 347-359.
McDougal, J. S., Mawle, A., Cort, S. P., Nicholson, J. K. A., Cross, G. D., Scheppler-Campbell, J. A., Hicks, D., and Sligh, J. (1985a). Cell tropism of the human retrovirus, HTLVIII/LAVI. Role of T cell activation and expression of the T4 antigen. J. Immunol. 135, 3151-3162.
McKnight, S. L. (1982). Functional relationships between transcriptional control signals of the thymidine kinase gene of herpes simplex virus. Cell 31, 355-365.
Muesing, M. A., Smith, D. H., and Capon D. J. (1987). Regulation of mRNA accumulation by a human immodeficiency virus trans-activator protein. Cell 48, 691-701.
Neville, D. M., and Hudson, T. H. (1986). Transmembrane transport of diphteria toxin, related toxins, and colicins. Ann. Rev. Biochem. 55, 195-224.
Page, K. A., Landau, N. R., and Littman, D. R. (1990). Construction and use of a human immunodeficiency virus vector for analysis of virus infectivity. Journal of Virology 64, 5270-5276.
Poznansky, M., Lever, A., Bergeron, L., Haseltine, W. and Sodroski, J. (1991). Gene transfer into human lymphocytes by a defective human immunodeficiency virus type 1 vector. Journal of Virology 65, 532-536.
Rosen, C. A., Sodroski, J. G., and Haseltine, W. A. (1985). The location of Cis-acting regulatory sequences in the human T cell lymphotropic virus type III (HTLV-III/LAV) long terminal repeat. Cell 41, 813-823.
Sczakiel, G., and Pawlita, M. (1991). Inhibition of human immunodeficiency virus type 1 replication in human T cells stably expressing antisense RNA. Journal of Virology 65, 468-472.
Sodrosky, J., Goh, W. C., Rosen, C., Campbell, K., and Haseltine, W. A. (1986). Role of the HTLV-III/LAV envelope in syncitium formation and cytopathicity. Nature 322, 470-474.
Sugden, B., Marsh, K., and Yates, J. (1985). A vector that replicates as a plasmid and can be efficiently selected in B-lymphoblasts transformed by Epstein-Barr virus. Molecular and Cellular Biology 5, 410-413.
Sullenger, B. A., Gallardo, H. F., Ungers, G. E., and Gilboa, E. (1990). Overexpression of TAR sequences renders cells resistant to human immunodeficiency virus replication. Cell 63, 601-608.
Trono, D., Feinberg, M. B., and Baltimore, D. (1989). HIV-1 gag mutants can dominantly interfere with the replication of the wild-type virus. Cell 59, 113-120.
Venkatesh, L. K., Arens, M. Q., Subramanian, T., and Chinnadurai, G. (1990). Selevtive induction of toxicity to human cells expressing human immunodeficiency virus type 1 tat by a conditionally cytotoxic adenovirus vector. Proc. Natl. Acad. Sci. USA 87, 8746-8750.

## Revendications

1. Association à des fins thérapeutiques:
(a) de cellules hématopoïétiques contenant une séquence génétique hétérologue codant pour une thymidine kinase sous le contrôle d'un promoteur assurant une expression basale non toxique pour l'hôte,
(b) de l'azido-thymidine (AZT) ou d'un analogue nucléosidique thérapeutiquement actif de l'AZT, et, le cas échéant,
(c) d'un médicament consistant en une molécule pharmaceutiquement active capable, lorsqu'elle se trouve associée in vivo aux cellules (a), d'interagir avec ladite thymidine kinase.

2. Utilisation de l'AZT ou d'un analogue nucléosidique thérapeutiquement actif de l'AZT, en association avec une population de cellules hématopoïétiques contenant une séquence génétique hétérologue codant pour une thymidine kinase sous le contrôle d'un promoteur assurant une expression basale non toxique pour l'hôte, pour la production de médicaments actifs contre une infection par des rétrovirus HIV.

3. Utilisation selon la revendication 2, dans laquelle le promoteur est constitué par un LTR de HIV, notamment un LTR partiel ou modifié.

4. Utilisation selon la revendication 2 ou 3, caractérisée en ce que la thymidine kinase est la thymidine kinase du virus de l'herpès simplex 1.

5. Utilisation selon la revendication 2, 3 ou 4, caractérisée en ce que l'analogue nucléosidique thérapeutiquement actif de l'AZT est choisi parmi la dideoxycytidine (DDC) et la dideoxynosine (DDI).

6. Utilisation de la thymidine kinase pour la préparation d'un médicament pour augmenter les propriétés antivirales de l'AZT ou d'un analogue nucléosidique thérapeutiquement actif de l'AZT.

## Claims

1. Combination for therapeutic purposes of :
(a) hematopoietic cells containing a heterologous genetic sequence coding for a thymidine kinase under the control of a promoter which ensures a basal expression non-toxic for the host,
(b) azido-thymidine (AZT) or a therapeutically active nucleoside analogue of AZT and, optionally,
(c) a medicine consisting of a pharmaceutically active molecule capable of interacting with said thymidine kinase when it is combined in vivo with the cells.

2. Use of AZT or a therapeutically active nucleoside analogue of AZT, in combination with a hematopoietic cell population containing a heterologous genetic sequence coding for a thymidine kinase under the control of a promoter which ensures a basal expression non-toxic for the host, for the production of medicines active against a HIV retroviral infection.

3. Use according to Claim 2, in which the promoter is constituted by an LTR of HIV, in particular a modified or partial LTR.

4. Use according to Claim 2 or 3, characterized in that the thymidine kinase is the thymidine kinase of the herpès simplex 1 virus.

5. Use according to Claim 2, 3 or 4, characterized in that the therapeutically active nucleoside analogue of AZT is selected from dideoxycytidine (DDC) and dideoxyinosine (DDI).

6. Use of the thymidine kinase for the preparation of a medicine to enhance the antiviral properties of AZT or a therapeutically active nucleoside analogue of AZT.

## Patentansprüche

1. Zusammenstellung zu therapeutischen Zwecken von:
(a) hämatopoietischen Zellen, die eine heterologe Gensequenz, die eine Thymidinkinase kodiert, unter der Kontrolle eines Promotors, der eine basale, für den Wirt nicht toxische Expression sicherstellt, enthalten,
(b) Azidothymidin (AZT) oder einem therapeutisch wirksamen Nukleosidanalog von AZT und gegebenenfalls
(c) einem Arzneimittel, das aus einem pharmazeutisch wirksamen Molekül besteht, das, wenn es in vivo mit den Zellen (a) assoziiert vorliegt, in der Lage ist, mit der Thymidinkinase zu interagieren.

2. Verwendung von AZT oder einem therapeutisch wirksamen Nukleosidanalog von AZT in Verbindung mit einer Population hämatopoietischer Zellen, die eine heterologe Gensequenz, die eine Thymidinkinase kodiert, unter der Kontrolle eines Promotors, der eine basale, für den Wirt nicht toxische Expression sicherstellt, enthalten, für die Herstellung von Arzneimitteln, die gegen eine Infektion durch HIV-Retroviren wirksam sind.

3. Verwendung nach Anspruch 2, bei der der Promotor durch einen LTR von HIV, insbesondere einen partiellen oder modifizierten LTR gebildet wird.

4. Verwendung nach Anspruch 2 oder 3, dadurch gekennzeichnet, daß die Thymidinkinase die Thymidinkinase des Herpes simplex-Virus 1 ist.

5. Verwendung nach Anspruch 2, 3 oder 4, dadurch gekennzeichnet, daß das therapeutisch wirksame Nukleosidanalog von AZT ausgewählt wird unter Didesoxycytidin (DDC) und Didesoxyinosin (DDI).

6. Verwendung der Thymidinkinase für die Herstellung eines Arzneimittels für die Erhöhung der antiviralen Eigenschaften von AZT oder einem therapeutisch wirksamen Nukleosidanalog von AZT.
